Europäisches Patentamt

(19) European Patent Office

Office européen des brevets

(11) Publication number: **0 048 422**
A2

# (12) EUROPEAN PATENT APPLICATION

(21) Application number: **81107234.7**

(22) Date of filing: **14.09.81**

(51) Int. Cl.³: **A 61 K 39/104**
**A 61 K 39/40**

(30) Priority: **15.09.80 US 187049**
**15.09.80 US 187050**

(43) Date of publication of application:
**31.03.82 Bulletin 82/13**

(84) Designated Contracting States:
**BE CH DE FR GB IT LI NL SE**

(71) Applicant: **BACTEX INCORPORATED**
**4307 Bigelow Blvd.**
**Pittsburgh, PA 15213(US)**

(72) Inventor: **Brinton, Charles C.**
**4307 Bigelow Blvd.**
**Pittsburgh, PA 15213(US)**

(74) Representative: **KUHNEN & WACKER**
**Patentanwaltsbüro**
**Schneggstrasse 3-5 Postfach 1729**
**D-8050 Freising(DE)**

(54) Pili of pseudomonas aeruginosa and utilization of same as vaccines against infectionary p.aeruginosa.

(57) There is provided a vaccine material capable of providing a substantial level of protection against infection by organisms of *Pseudomonas aeruginosa*. The protecting means comprises pili of the infecting organism. The protection is given either by administering the pili directly to the subject to be protected or by the passive administration of immune serum from another subject to whom *P. aeruginosa* pili have been previously administered.

The material, or its immune serum, is effective in protecting burned subjects against *P. aeruginosa* infection.

FIGURE I

WAVELENGTH (NM.)

## BACKGROUND OF THE INVENTION

Pseudomonas aeruginosa is a widespread pathogenic organism. It is particularly prevalent as the infecting organism in burn cases. It is also prevalent as the infecting organism in cancer patients, wounds, cystic fibrosis cases, the elderly, the very young and traumatized patients generally. It is a commonly acquired hospital infection. In view of the weakened condition of many patients, normal antibiotic therapy is often not practical. Thus, it would be most useful to provide a material administrable as late as occurrence of the onset of infection which would inhibit spread of infection due to this organism.

## SUMMARY OF THE INVENTION

There is provided a material derived from pili of piliated organisms of Pseudomonas aeruginosa. This material is substantially free of cell material or cell debris and is isolable, if desired, in crystalline form.

There is provided further a vaccine composition utilizing the aforesaid material capable of raising the antibody level of a vertebrate subject to a level sufficient to provide protection against infection caused by organisms of a first group of strains of piliated P. aeruginosa comprising:

    a)   Pili derived from a second group of strains of piliated P. aeruginosa organisms wherein cells of organisms of said first group are cross-reactive with serum containing antibodies against pili from said second group, said first group consisting of strains which may be the same or different from the strains of said second group; and,

b) a pharmaceutically acceptable carrier.

There is exemplified in this application a vaccine containing pili from a single predetermined strain which is effective against infection caused by organisms of the same strain, vaccines which contain pili from several different strains are included within the scope of the present invention provided, of course, that each of said pili will cross-react immunologically with at least one of the infecting organisms.

An important pathogenic strain of P. aeruginosa was isolated from the urine of a burn patient shown to be virulent and causative of the natural disease when inoculated into non immune mice. This virulent strain was grown and selected for well-piliated clones. These clones were grown and maintained on tryptosebroth agar medium. The clones are suitably grown on a medium comprising casamino acids, yeast extract and agar. The cells are harvested in a suitable buffer, blended and cell debris removed by centrifugation. The supernate is concentrated with polyethylene glycol which produces crystallization of the pili, these are then separated by centrifugation and purified by sequential solubilization and crystallization.

The materials were injected into mice. The mice were challenged with ethanol burns and the piliated organisms of P. aeruginosa injected subcutaneously under the burn. The immunized mice developed bacteraemia and died between 20 and 90 hours after challenge. Immunized mice receiving between 5 ug/kg and 0.5 mg/kg of pili were substantially immune to infection.

DESCRIPTION OF THE PREFERRED EMBODIMENT

The vaccine compositions of the present invention comprise pili of a predetermined strain which pili meet certain criteria.

It is known that P. aeruginosa organisms carry a number of antigenic factors known as "O" antigens (outer membrane) "K" (capsule), "H" (flagella), and the like, as well as, for certain strains, pili. The criterion of the strain of organisms selected shall be that the strain to be protected against shall be piliated, that the protecting strain shall be piliated, and that the pili of one strain shall give rise to antibodies which cross-react with pili of the other strains. This simple criterion means that the protecting strains may be homologous or heterologous with respect to the infecting strain as long as the pili of each are immunologically similar. This similarity may, as above, be readily determined by one skilled in the art without undue experimentation.

It has been found that a well definable hierarchial relationship can be provided between all members of the immunologically cross-reactive piliated organisms, that is to say, where organisms are piliated and the pili thereof may be isolated and purified and utilized to form antisera containing antibodies to said pili, the organisms may be arranged in a hierarchial order. It has been the surprising finding of the present work that within such an immunologically cross-reactive group the degree of cross-reactivity between the antiserum of a first strain and the pili of a second strain in the group will not be the same as the degree of cross-reactivity between the antiserum to the pili of said second strain with the pili causing the antibodies to said first strain.

This surprising difference has led to a mode of ranking members of an immunological group in a clearly defined hierarchy.  In carrying out this method, pili of the group of piliated organisms are prepared free of cell material and cell debris. The pili are then provided to an immune responsive system, such as, for example, mice or rabbits, and the antisera to said pili containing the antibodies to said pili are then isolated in the usual manner.

Cross-reaction experiments of the type well known to the art, for example, ELISA Tests, are carried out between the pili and the antisera.  Suitably, these results are translated on a table in matrix form showing the antisera on the columns and the corresponding pili on the rows. Such a classification will provide the homologous reaction readings between a particular pilus and the antiserum corresponding thereto upon the diagonal.  The diagonal numbers are then normalized to the same predetermined value and the corresponding titers for all heterologous pili against each antiserum adjusted accordingly. The resultant Table will indicate the hierarchial relationship.

Upon rearrangement of this normalized Table, in the order of seniority, it will be seen that a remarkable but consistent asymmetry exists between the titers of the antisera with the pili.  The titers obtained between the antiserum to the pili of a more senior strain and the pili of a more junior strain is always greater than the titer obtained between the pili of the said more senior strain with the antiserum to the pili of the said more junior strain.

This relationship holds within families of immunologically related pili.

This remarkable result has led to the conclusion
that within any such group the senior organisms
contain an immunological factor which will provide
an immune response against all organisms in the
hierarchy equal in rank or junior toit, thus providing
a vaccine inducing protection against infection
therefrom. Conversely, the juniormost organisms
will contain a factor common to all organisms senior
to them in the hierarchy, thus enabline the juniormost
organism to be utilized for the purpose of detecting
antibodies to members of the family senior thereto.

Hence with respect to P. aeruginosa vaccines,
compositions derived from senior strains will protect
against infections by junior strains.

The pili are selected and obtained by methods
well known in the art. One important disease preventable
by the methods of the present invention is burn
infection. This disease is caused by P. aeruginosa
infection. One infecting strain has been found to
be P. aeruginosa 577B (ATCC        ).

Culture of P. aeruginosa 577B

Samples of a parental strain P. aeruginosa 577B
were isolated from a burn patient suffering from
P. aeruginosa infection, passed through still broth,
and colonial forms selected therefrom, to provide
a well piliated clone designated P. aeruginosa (ATCC
       ). The clone is initially grown on tryptose broth
agar medium and grown on casamino acid/yeast extract
agar. The pili were separated from the cells by
blending and centrifugation in a low ionic strength
neutral buffer such as 0.001 ionic strength pH 7.0 phosphate buffer. The pili were crystallized from the buffer by addition thereto of polyethylene glycol (Carbowax 6000) (PEG) to bring the concentration of the buffer up to 3% PEG whereupon the pili crystallize. The crystalline pili were taken up in a low ionic strength neutral buffer such as 0.001 ionic strength pH 7.0 phosphate buffer and reprecipitated with PEG in a similar manner. It is preferred to subject the pili to from one to five cycles of recrystallization.

The final preparation of the pilus vaccine consists of diluting the recrystallized pili into saline, suitably saline containing phosphate buffer as above. The pili thus prepared are of a quality sufficient to pass the standards of the Bureau of Biologics, Food and DrugAdministration, for general safety, sterility, and pyrogenicity.

The pili may be administered parentarally, that is to say, by subcutaneous, intradermal, or intramuscular injections. Since the pili are solid, any pharmaceutically acceptable suspending medium may be employed. It has been found especially useful to employ saline or phosphate buffered saline, suitably containing formaldehyde, as the vehicle or suspending medium. It is preferred to use 0.7 - 0.9, most suitably 0.85%, saline containing 0.01 to 0.1, most suitably 0.05%, formaldehyde. The concentration of pili in the vehicle is not critical. The sole criterion of desirability being that the pili shall be sufficiently finely divided to provide a suspension which meets generally accepted standards of syringeability. A concentration of 0.1 - 1, preferably about 0.5 mg of pilus protein per ml of suspending medium is especially suitable.

It is generally preferred to administer the vaccine composition in more than one dose separated by a predetermined time factor. This time factor is selected to permit the formation of an adequate titer of antibodies to the pili in the injected subject.

Since there are no local or systemic toxic effects engendered by the injection of vaccine, there appear to be no upper limits to the dosage administered. It has been found suitable, however, to administer between 1 and 1000 micrograms of pili per kilogram of body weight, most suitably about 500 micrograms per kilogram of body weight in each injection. While it is preferable that immunization occurs in advance of exposure to infection, it has been found that immunization immediately after burn occurrence will offer protection.

Under certain acute conditions an infected or potentially infected system is either unable to generate antibodies to the pili to provide the necessary protection, or is not able to do so rapidly enough. Under these circumstances, it is advantageous to administer antibodies directly. The antibodies may be provided by previously injecting subjects of the same species with pili from a predetermmined strain or strains, permitting the subject system to generate the appropriate antibodies and then removing blood fluids containing said antibodies. In the preferred system the blood fluids are centrifuged to remove the redcells (which may, if desired, be returned to the donor). The residual serum is then treated by methods known in the art to remove factors which would cause negative reactions in the donee, and the residual serum, containing the antibodies to the infecting organism, is administered parenterally to the subject in need of same.

## EXAMPLE I

### Preparation of Pseudomonus aeruginosa

Bacteria from a 5 day still culture of P. aeruginosa 577B in Z broth (1% Bactotryptone, 0.8% NaCl, 0.1% Yeast Extract, 0.1% glucose) were diluted and plated on Tryptose Broth + 1.8% agar medium. The plates were incubated for 20 h at 37°C and piliated colony types, selected by morphology, were resuspended in 0.85% sterile saline to a concentration of $10^8$ cells/ml. Piliated colony types of 577B were maintained on Tryptose Broth (Difco) + 2.0% agar (tryptose broth agar). Five petri dishes containing CAYE (0.75% CAA- casaminoacids, 0.15% Yeast Extract and 2.0% agar) were heavily streaked with 577B and allowed to incubate for 15 h at 37°C. Growth from the plates were suspended in 0.85% NaCl (sterile) to an approximate concentration of $10^9$ cells/ml. Pyrex baking trays with aluminum lids containing CAYE medium were inoculated with 3 ml of the cell suspension. The trays were incubated at 37°C for 14 h. Trays were harvested with phosphate buffer pH 7.0, 0.01u and the suspension was subsequently blended in 100 ml volumes at 5,000 rpm for 5 minutes in a Sorvall Omnimixer. Cell debris was removed by centrifugation at 13,200 x g twice. The extract was then brought to a final concentration of 3% PEG (Carbowax 6000). Under these conditions pili crystallized. The pili were sedimented by centrifugation at 27, 300 x g for 1 h. The pellets were resuspended in solubilizing buffer and recycled by alternating crystallization and solubilization steps until a single band appeared on SDS-polyacrylamide gels.  All centrifugations
were carried out at 4°C.  Pili were stored at 4°C
phosphate buffer pH 7.0, 0.01 ionic strength.

In, accordance with the above procedure P.
aeruginosa 653A (ATCC 31707), P. aeruginosa PSA-T2A
(ATCC 31708) and P. aeruginosa 1244WR (ATCC 31710)
may similarly be grown and their pili isolated.

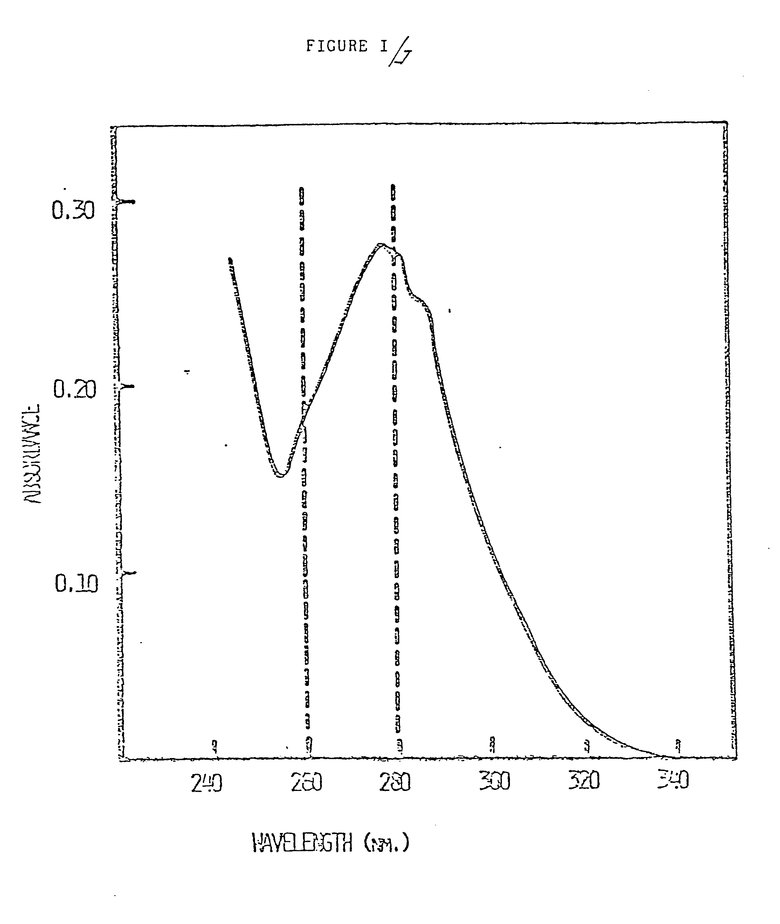

One criterion for purity and homogeneity of
our pilus preparations is SDS-polyacrylamide gel
electrophoresis.  The apparent gel molecular weight
of the pili is about 18,000 daltons.  Figure 1
shows the characteristic ultraviolet absorption
spectrum of 577B pili which is also used as a
criterion of purity.  The diameter of these pili
is 5.7 mm as determined from electron micrographs.
The amino acid composition of 577B pili is shown
in Table I below and is characteristic for this
particular kind of pili.

TABLE I

| Amino Acid | Number of Residues |
|---|---|
| 1/2 cysteine | 2 |
| Aspartic acid | 16 |
| Threonine | 19 |
| Serine | 15 |
| Glutamic acid | 18 |
| Proline | 6 |
| Glycine | 19 |
| Alamine | 20 |
| Valine | 14 |
| Methionine | 1 |
| Isoleucine | 16 |
| Leucine | 12 |
| Tyrosine | 4 |
| Tryptophan | 1 |
| Phenylalanine | 2 |
| Histidine | 0 |
| Lysine | 12 |
| Arginine | 3 |

The subunit molecular weight calculated from these values is 18,485 daltons.

0048422

## EXAMPLE II

Mode of Immunization

The vaccine and the mode of protecting burned mice was evaluated with groups of mice. Ten mice were injected with pili and ten with vehicle. The vehicle utilized phosphate-buffered (0.001 ionic strength, pH 7.0) saline (0.85%) and Freund's Incomplete (mineral oil) adjuvant. The vaccine consisted of vehicle containing 0.5 mg of pili per ml of vehicle. P. aeruginosa 577B pili were used. Each vaccinated mouse received between approximately 0.1 and 10 of pili per mouse or a dosage circa 4 and 400 micrograms per kilogram per injection. Three injections were given subcutaneously behind the neck. Injections were given on days 0, 5 and 13 and mice were challenged on day 19 or 20. No local or systemic toxic effects were noted.

## EXAMPLE III

Results of 577B pilus immunization

The results of immunization of mice at various doses of 577B pili are shown in Table 2. The pili were antigenic as measured by an enzyme-linked immunosorbent assay. At a dose of 10 miligrams pili per mouse, the pilus semi-antibody level was raised approximately 200-fold above the pre-immunization level.

0048422

## TABLE 2

Antigenicity/Immunogenicity Dose-Response
Experiments:

| Immunization µg pili/mouse | Antibody Titer = Standard Dev. | Fraction Subjects w/ Bacteremia & Death |
|---|---|---|
| 10.0 | 1033 ± 465 | 0.05 |
| 1.0 | 70 ± 98 | 0.10 |
| 0.1 | 7.2 ± 11 | 0.31 |
| 0.0 | 0.5 ± 2 | 0.79 |

Grouped Data:

| Group No. | Titer Range | Avg. Antibody Titer ± Standard Deviation | Fraction Sub. w/ Bacteremia & Death |
|---|---|---|---|
| 1 | 800 | 1435 ± 188 | 0.07 |
| 2 | 90 - 800 | 345 ± 41 | 0.15 |
| 3 | 10 - 90 | 30 ± 7.2 | 0.41 |
| 4 | 0 - 10 | 0 ± 0 | 0.63 |

## EXAMPLE IV

Preparation of Inocula for P. aeruginosa 577B

Inocula. Frozen samples of 577B selected for virulence were thawed and added to 100 ml of Z medium. The cultures were incubated with shaking for 5 days at 37°C, diluted and plated on Brain Heart Infusion Agar. The plates were incubated for 18 to 20 hours. Inocula were prepared by touching many $P^+$ or $P^-$ colonies with a sterile swab and resuspending cells in sterile saline to an optical density at 610 nm of 0.3 (approximately $5 \times 10^8$ cells/ml). This was diluted in sterile saline to the appropriate concentrations. Samples were plated for viable counts. All inocula were prepared in sterile tubes (Vacutainer; Becton Dickinson Co.) and held at 35°C until injection into mice. New inocula were prepared every hour and coded.

## EXAMPLE V

Challenge

Mice were anaethetized with 0.35 ml of 5% penta-barbitol and prepared for challenge in groups of 10. After 2 to 3 minutes, their backs were shaved with electric clippers and dipilatory was brushed onto the shaved portion. After 2 minutes the depilatory was washed off and the mice were dried. An asbestos board with a 1" x 1.5" window was pressed firmly over the shaved back. 0.5 ml of 95% Ethanol was evenly spread over the area of the back exposed by the window, ignited and allowed to burn for 20 seconds.

The flame was blotted out with a piece of teflon. Immediately thereafter the animals were injected subcutaneously at the burn site with appropriate concentrations of 577B in 0.5 ml of saline. Experiments were performed double-blind.

Mice were observed for 4 days; every hour at first and then every 6 to 8 hours. They were observed for neurological signs of bacterémic toxicity and death. Dead mice were opened aseptically immediately after death, their hearts were removed and each heart was placed in 1 ml of sterile saline. This was vortexed for 20 to 30 seconds at high speed, and the supernatant cultured on tryptose agar.

Results of 577B Challenge

The results of challenge as a function of infectious dose are shown in Table 3. The immunized mice were essentially completely protected from bacteremia and death at doses of the infecting organism ranging from $4.3 \times 10^3$ to $1.0 \times 10^7$. These doses resulted in bacteremia and death of from 20% to 72% of non-immunized mice.

## TABLE 3

### Infective Dose-Response/ActiveImmunization
### Experiments

| Experiment Number | # Mice | Challenge dose organisms/mouse | Variant ($P^+$ - | Fraction Response (death and culture +) | Immunization ,S,or I |
|---|---|---|---|---|---|
| 1 | 10 | $4.3 \times 10^3$ | $P^+$ | 0/10 | I |
|   | 10 | $4.3 \times 10^3$ | $P^+$ | 5/10 | S |
| 2 | 10 | $5.2 \times 10^3$ | $P^+$ | 0/10 | I |
|   | 10 | $5.2 \times 10^3$ | $P^+$ | 2/10 | S |
| 3 | 11 | $2.0 \times 10^5$ | $P^+$ | 1/11 | I |
|   | 19 | $2.0 \times 10^5$ | $P^+$ | 11/19 | S |
| 4 | 19 | $1.4 \times 10^5$ | $P^+$ | 1/19 | I |
|   | 20 | $1.4 \times 10^5$ | $P^+$ | 12/20 | S |
| 5 | 15 | $1.0 \times 10^7$ | $P^+$ | 0/15 | I |
|   | 18 | $1.0 \times 10^7$ | $P^+$ | 13/18 | S |

I:  Immunized

S:  Sham immunization

## EXAMPLE VI

Cross-reactivity

The pili from two independently isolated pathogenic strains of P. aeruginosa were isolated, purified and injected into rabbits. The resulting antisera specifically agglutinated pilis crystals of the homologous strain. These two antisera were used to measure the cross-reactivity of the pili of eleven additional strains. The method of measurement was agglutination of piliated bacteria. The results are shown in Table 4. The pili of 5 of 11 additional strains (45%) cross-reacted to a significant degree with P. aeurginosa 577B or 686 pili.

0048422

## Table 4

SEROLOGICAL SCREENING OF <u>PSEUDOMONAS</u> <u>AERUGINOSA</u>
ISOLATES WITH SPECIFIC ANTIPILUS ANTISERUM

| STRAINS | Antipilus Antiserum | |
|---|---|---|
| | 577Bp$^+$ | 686B |
| PAO38 | − | − |
| 93F | ++ | − |
| 149S | +++ | − |
| 577Bp$^+$ | ++++ | +/− |
| 631S | +++ | − |
| 653A | +/− | − |
| 656R | +/− | ++ |
| 686B | +/− | ++++ |
| 716M | +/− | − |
| 732P | +/− | − |
| P2004S | +++ | − |
| P2005M | +/− | +/− |
| 1244 (McManus) | +/− | − |

## CLAIMS

1.　A material derived from pili of P. aeruginosa which is substantially free of cells or cell debris which shows strong cross-reactivity against sera containing antibodies against pili derived from P. aeruginosa.

2. A material of Claim 1 comprising pili derived from at least one member of a second group of strains piliated P. aeruginosa organisms wherein cells of organisms of said first group react with serum containing antibodies against pili from said second group, said first group consisting of strains which may be the same as or different from the strains of said second group.

3.　A material of Claim 2 comprising pili derived from more than one member of the second group of strains wherein each of said members of said second group are capable of producing antibodies which will react with at least one member of said first group.

4.　A material of Claim 2 wherein a strain of the second group is P. aeruginosa 577 B (ATCC 31706).

5.　A vaccine composition capable of raising the antibody level of a vertebrate subject to a level sufficient to provide protection against infection caused by a member of a first group of strains of piliated P. aeruginosa comprising a material of Claim 1 and a pharmaceutically acceptable carrier.

6.　A vaccine composition capable of raising the antibody level of a vertebrate subject to a level sufficient to provide protection against infection caused by a member of a first group of strains of
piliated P. aeruginosa comprising

      a)    pili derivedfrom at least one member
             of a second group of strains of piliated
             P. aeruginosa organisms wherein cells
             of organisms of said first group are
             agglutinable by serum containing antibodies against pili from said second
             group, said first group consisting
             of strains which may be the same as
             or different from the strains of said
             second group, and
      b)    a pharmaceutically acceptable carrier.

7. A composition of Claim 6 comprising 1-10
mg of pili of the second group per 10 ml of injectable
vechile.

8. A composition of Claim 6 wherein the pilic
component of the vaccine composition is derived from
at least one member of the group of pili of P.
aeruginosa 577 B (ATCC 31709), 1244 (ATCC 31710),
686 (ATCC 31711), 653A (ATCC 31707), or T2A (ATCC
31708).

9. Species homologous serum compositions comprising antibodies to P. aeruginosa produced by providing
pili substantially free of cell material or cell
debris, derived from P. aeruginosa into a living
system of a predetermined species, permitting said
system to generate antibodies to said pili, removing
blood fluids from said system and separating the serum
components containing said antibodies from said blood
fluids.

10. A vaccine composition of Claim 2 wherein the strains of the said first group are the same as or junior to the strains of said second group.

FIGURE I

WAVELENGTH (nm.)